# FASCICULE DE BREVET EUROPEEN

(11) **EP 2 644 159 B1**
(45) Date de publication et mention de la délivrance du brevet: **12.10.2016**
(21) Numéro de dépôt: 13161403.4
(22) Date de dépôt: 27.03.2013
(51) Int. Cl.: A61F 2/00

(54) **PLAQUE VISCÉRALE DESTINÉE À LA COELIOSCOPIE**
Viszerale Platte für Zölioskopie
Visceral plate for laparoscopy

(30) Priorité: 28.03.2012 FR 1252780
(43) Date de publication de la demande: 02.10.2013
(73) Titulaire: Cousin Biotech, 59117 Wervicq Sud (FR)
(72) Inventeur: Noel, Stéphane, 59496 Hantay (FR); Solecki, Gilles, 59390 Lannoy (FR); Barlian, Mathilde, 59350 Saint André lez Lille (FR)
(74) Mandataire: Balesta, Pierre

(56) Documents cités:
- EP-A2- 1 634 609
- WO-A1-2004/103187
- WO-A1-2012/034126
- WO-A2-2007/056297
- US-A1- 2010 189 764

## Description

La présente invention concerne le domaine des implants textiles avec des monofilaments en particulier de polypropylène, notamment pour la réparation pariétale ou du prolapsus par voie haute par voie coelioscopie.

La technique de chirurgie par coelioscopie permet de poser une plaque de hernie ou d'éventration à l'aide de trocarts, de deux pinces et à l'aide d'une caméra endoscopique qui traverse la paroi abdominale. Pour l'introduction dans la cavité abdominale, le chirurgien doit enrouler la plaque, initialement dans une conformation plane, pour la faire passer ensuite dans l'un des trocarts qui traversent la paroi abdominale. La plaque se déploie ensuite d'elle-même du fait de son élasticité en flexion à la sortie du trocart pour retrouver sa forme plane. Le chirurgien peut, de ce fait, la fixer facilement à la paroi dans cette conformation déployée. Cette élasticité naturelle en flexion est en général obtenue par utilisation dans la structure du textile tricotée d'un monofilament en polypropylène de diamètre supérieure à 0,1 mm et sous condition d'avoir une plaque de poids supérieur à 50 g/m². Il est par contre impossible de poser une plaque de poids inférieur à 50 g/m² même si elle est fabriquée avec un monofilament de polypropylène supérieur à 0,1 mm. En effet, elle n'aurait pas assez d'élasticité en flexion pour retrouver sa forme plane à la sortie du trocart.

Il n'est pas envisageable pour l'homme de la technique (le fabriquant) de livrer une plaque de poids supérieur à 50 g/m² déjà roulée dans un emballage ou un tube. En effet, la plaque garderait une mémoire élastique de cette forme enroulée après une période de stockage courte à température ambiante. Autrement dit, si cette plaque préroulée était introduite dans la cavité abdominale, le chirurgien serait incapable de redonner une forme plane sans exercer un effort important à l'aide de pinces célioscopiques. En outre, si cet effort exercé pour la dérouler était supprimé, la plaque reprendrait sa forme roulée initiale de façon spontanée. Ce phénomène s'explique par le comportement plastique des polymères qui ont tendance à garder une forme donnée lorsque l'on exerce une contrainte pendant une durée importante. Ce phénomène dit de fluage est une des particularités des polymères.

Pour des plaques légères en polypropylène de poids inférieur à 50 g/m², ce phénomène de fluage existe mais il est négligeable. En effet le problème principal vient du fait que les plaques ne se déploient pas d'elles-mêmes, qu'elles aient été stockées enroulées ou non. Ainsi, le chirurgien peut déployer la plaque même si elle a été longtemps stockée enroulée (car la mémoire de forme de la forme enroulée est faible pour des poids de plaque inférieur à 50 g/m²), mais il est contraint de la dérouler avec les pinces à la sortie du trocart. Cette manipulation est très difficile à faire en coelioscopie et augmente de façon importante le temps de l'opération chirurgicale.

Dans l'art antérieur, il est proposé pour palier à ces problèmes et uniquement pour des plaques tricotées en polypropylène de poids supérieur à 50 g/m² des enrouleurs de plaques mis à la disposition du chirurgien afin d'enrouler les plaques juste avant l'opération pour éviter les problèmes de fluages (EP 0.625.334, EP 0.535.506 et WO 92/06638). Toutefois, cela oblige le chirurgien à une manipulation supplémentaire avant implantation et cela ne règle pas le problème des plaques de poids inférieur à 50 g/m².

Il est également proposé par la demande de brevet FR 2.771.622 un dispositif pour la mise en place de prothèse par voie coelioscopie comprenant un introducteur constitué d'un tube cylindrique pouvant contenir la prothèse, d'un piston pour l'injection de la prothèse et d'un ressort solidarisé avec le piston et solidarisé de façon amovible avec la prothèse qui permettra le déploiement de la prothèse à l'extérieur du tube. Malheureusement ce dispositif n'est pas adapté à toutes les prothèses puisqu'il faut que la prothèse soit pourvue de moyens pour recevoir les ressorts. En outre, ce dispositif nécessite que le chirurgien apprenne à se servir du ressort permettant de dérouler la plaque de la maintenir.

US 2010/0189764 A1 propose une plaque pour le traitement de la hernie ayant une masse surfacique résiduelle de 50 g/m² ou moins (paragraphe 50), i.e la masse de la plaque subsistant dans l'organisme une fois celle-ci implantée et les composants résorbables la constituant absorbés. La plaque peut ainsi comprendre des fibres bicomposantes dont le manteau est dans un matériau résorbable (voir figure 8d). La plaque est ainsi rigide avant son implantation, ce qui lui permet de retrouver sa forme géométrique originale une fois déployée (fin paragraphe 188, revendication 1). Du fait de la résorption de la plaque une fois implantée, les risques d'érosion et d'inconfort susceptibles d'être provoquées par sa rigidité initiale sont limités (voir paragraphes 11 et 12). La plaque décrite dans US 2010/0189764 A1 se comporte ainsi telle une plaque ayant une masse surfacique avant son implantation supérieure à 50 g/m² et utilisant sa rigidité en tant que moyen de déploiement.

WO 2012/035426 A1 a pour objet un textile médical, par exemple en polypropylène, intégrant un polymère à mémoire de forme, ledit textile pouvant être utilisé comme plaque pour le traitement d'une hernie (voir paragraphe 7). WO 2012/035426 A1 ne décrit ni la structure du fil employé dans la plaque, ni la masse surfacique de la plaque mais énumère de nombreux polymères à mémoire de forme. Généralement, les matériaux à mémoire de forme sont utilisés en combinaison avec une plaque comme moyen de déploiement afin que ladite plaque, à l'état enroulé dans le dispositif d'introduction sur le site d'implantation, retrouve sa configuration initiale une fois libérée dudit dispositif, i.e sa configuration parfaitement plane au repos lors de son stockage.

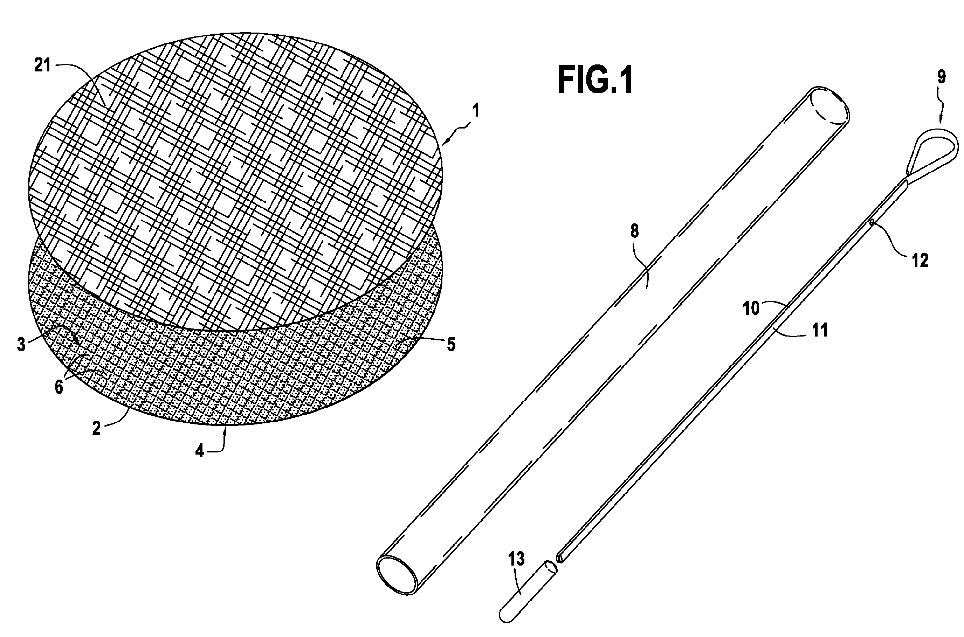

WO 2012/035426 n'aborde pas le problème qu'une plaque puisse retrouver sa structure plane après avoir été stockée à l'état enroulé sur elle-même dans une configuration de tube pendant une période prolongée. D'ailleurs, tel que cela est visible à la figure 1A, la plaque textile 104 revêtues selon ses deux faces d'un matériau polymère à mémoire de forme 102 est en sandwich entre deux plaques de verre à travers lesquelles des rayons UV sont transmis pour la photopolymérisation des matériaux 102. La plaque 104 en sortie du procédé adopte ainsi une configuration plane.

Ainsi pour ces différentes raisons, aucune plaque en monofilament de polypropylène ne peut être livrée dans une forme enroulée, que la plaque ait un poids inférieur ou supérieur à 50 g/m². En outre, les plaques de poids inférieur à 50 g/m² sont difficilement implantables car elles ne se déploient pas d'elles-mêmes. Ainsi donc il existe un besoin de pouvoir fournir au chirurgien des plaques pré-enroulées qui se déploient spontanément d'elles-mêmes après implantation, sans avoir besoin de manipulation supplémentaire de la part du chirurgien.

Les inventeurs ont découvert de façon surprenante qu'il était possible de répondre à ce besoin à l'aide d'une plaque textile de masse surfacique inférieure à 50 g/m² enduite d'un polymère sensible à l'humidité et avantageusement à la chaleur. Avant implantation la plaque textile enduite se trouve sous une forme pré-enroulée car le polymère est déshydraté. Après implantation, le polymère se transforme en gel lors de son contact avec le milieu humide du site d'implantation et à la température corporelle (37°C), ce qui permet à la plaque textile de se déployer pour avoir une configuration sensiblement plane.

Des plaques enduites par des polymères sensibles à l'humidité telle que des polymères bio-adhésifs dont la fonction adhésive est activée par le milieu aqueux d'implantation sont déjà connues (FR 2.920.671 et WO 2005/058383). Toutefois, ces plaques enduites avaient au repos une configuration sensiblement plane puisque la composition bio-adhésive était suffisamment rigide pour conférer à l'implant textile une bonne mémoire de forme ce qui permettait à ce dernier de se déployer facilement et totalement à la sortie du trocart. Ainsi, le chirurgien devait enrouler lui-même la plaque juste avant implantation. Or, de façon surprenante, les inventeurs ont découvert que si, après enduction et séchage des polymères bio-adhésifs, un tel implant, lorsqu'il a une masse surfacique inférieure à 50 g/m², est maintenu sous une forme enroulée sur lui-même pendant un temps suffisamment long, il garde au repos cette configuration enroulée sans déploiement, même lorsque le dispositif n'est plus maintenu dans cette configuration. Les inventeurs ont également découvert qu'en revanche après implantation lorsque le polymère est mis en contact avec le milieu aqueux, le poids du gel obtenu provoque le déploiement spontané de la plaque sans aide supplémentaire.

Il est également déjà connu d'utiliser des plaques enduites par des polymères sensibles à l'humidité telle que le collagène ou les dérivés de l'acide hyaluronique afin d'obtenir un film permettant d'éviter d'adhérence après implantation (WO 96/08277 et WO 99/06079). Toutefois ces plaques sont hydratées avant implantation par le chirurgien afin de préserver leur maniabilité, par trempage dans une solution physiologiquement compatible telle que le sérum physiologique. Le gel se forme donc avant implantation et la plaque doit être ensuite enroulée sur elle-même par le chirurgien juste avant son implantation. Ceci nécessite donc beaucoup de manipulation de la part du chirurgien et des risques de contamination du gel lors de son hydratation. Or de façon surprenante les inventeurs ont découvert qu'il n'était pas nécessaire d'hydrater le polymère sensible à humidité avant implantation et qu'au contraire il était plus avantageux de laisser le milieu d'implantation hydrater le polymère afin qu'il se transforme en gel in situ. En effet ceci permet d'utiliser une plaque préenroulée et d'obtenir son déploiement in situ grâce à la formation de ce gel.

La présente invention concerne donc un dispositif implantable, tel que décrit dans les revendications, notamment par coelioscopie, comprenant une plaque textile ayant des faces envers et endroit opposées, une masse surfacique inférieure ou égale à 50 g/m², avantageusement inférieure ou égale à 40 g/m², en particulier inférieur ou égale 30 g/m², plus particulièrement supérieure ou égale à 10 g/m², encore plus particulièrement supérieure ou égale à 20 g/m², et comprenant des monofilaments, ladite plaque se présentant dans une position au repos enroulée sur elle-même dans une configuration de tube, comprenant des moyens de déploiement activables par milieu aqueux aptes à faire passer ladite plaque de ladite position au repos à une position d'implantation dans laquelle ladite plaque a une configuration sensiblement plane.

Les moyens de déploiement sont disposés sur la face envers et/ou la face endroit de la plaque et comprennent un polymère déshydraté apte à former un gel en milieu aqueux.

Au sens de la présente invention, on entend par « position au repos », la configuration que prend la plaque spontanément, sans aucun moyen de contrainte lorsque les moyens de déploiement ne sont pas activés. Ainsi, dans le cadre de la présente invention, le dispositif implantable présente une configuration de tube dans sa position au repos. En conséquence, même si on le contraint à modifier cette configuration, par exemple en le déroulant à l'aide d'instruments chirurgicaux, il reprendra spontanément sa configuration de tube dès que les contraintes auront cessés, à la condition qu'il n'ait pas été mis en contact avec un milieu aqueux et que les moyens de déploiement n'aient donc pas été activés.

Au sens de la présente invention, on entend par « position d'implantation », la configuration que prend la plaque lors de son implantation dans le corps humain. Lors de son implantation, la plaque est mise en contact avec le milieu aqueux du corps humain (sang, etc..) ce qui va provoquer l'activation des moyens de déploiement est donc le passage de la configuration de tube qu'à la plaque au repos à la configuration sensiblement plane, qui correspond donc à la position d'implantation. En effet, pour pouvoir implanter cette plaque, il est nécessaire qu'elle soit déroulée et qu'elle ait donc cette configuration sensiblement plane afin que le chirurgien puisse la suturer ou la coller à la paroi abdominale par exemple.

Avantageusement, la plaque textile selon la présente invention est en tissu tissé, tricoté ou non-tissé. De façon encore plus avantageuse, la plaque textile est un tricot, en particulier du type chaîne ou raschel.

Dans un mode de réalisation avantageux, la plaque textile selon la présente invention comprend des orifices ayant une forme géométrique déterminée, notamment une forme de losange, rectangle, carré, hexagonale, ou octogonale, avantageusement une forme de losange, délimitée par des segments constitués de fils tricotés. Avantageusement les droites qui prolongent lesdits segments sont sécantes à la direction d'enroulement (P) de ladite plaque. En effet, ceci évite que la plaque possède une mémoire de forme trop importante.

De façon avantageuse, la porosité est comprise entre 0,5 et 4 mm, de façon encore plus avantageuse entre 0,5 et 2 mm, en particulier de l'ordre du millimètre. Ces orifices peuvent traverser totalement ou non l'épaisseur de la plaque textile selon la présente invention. Plus avantageusement, ces orifices la traversent totalement et la plaque textile a donc une structure ajourée. Ainsi la plaque textile peut être constituée par une multiplicité d'alvéoles ou canaux transversaux sensiblement parallèles les uns aux autres. L'avantage d'avoir une plaque textile ajourée est de favoriser l'accroche des tissus conjonctifs développés suite à la fibrose sur les mailles délimitant les jours de ladite plaque textile.

Dans un autre mode de réalisation avantageux de la présente invention, la plaque textile selon la présente invention est tricotée avec des monofilaments, de préférence ayant un diamètre inférieur ou égal à 0,2 mm, de façon préférentielle inférieur ou égal 0,1 mm, en particulier de l'ordre de 0,1 mm.

Dans un mode particulier réalisation de la présente invention, la plaque textile selon la présente invention est en polymère biocompatible flexible et est non résorbable, c'est-à-dire qu'elle reste de façon permanente dans le corps, ou semi-résorbable, c'est-à-dire que seulement une partie de la plaque textile reste de façon permanente dans le corps. La plaque semi-résorbable est fabriquée avec des fils en polymère résorbable et des fils en polymère non résorbable. Avantageusement le polymère est un matériau synthétique ou semi-synthétique. De façon avantageuse, dans le cas où il est non résorbable, il est choisi parmi les polyéthylènes, polyesters, polypropylène, polytétrafluoroéthylène (PTFE), polyamide (6,6), polypropylène/PTFE, polyéthylène téréphtalate. En particulier il s'agit du polypropylène. Dans le cas où la plaque textile est semi résorbable, les fils en polymère résorbable se trouvent sous la forme de polymère d'acide lactique de forme D ou L (PLLA ou PDLA) ou de copolymère d'acide lactique et glycolique (PLGA) tandis que les fils en polymère non résorbable sont choisis parmi les polyéthylènes, polyesters, polypropylène, polytétrafluoroéthylène (PTFE), polyamide (6,6), polypropylène/ PTFE, polyéthylène téréphtalate. En particulier par exemple la plaque semi-résorbable est composée de fils résorbable en PLLA de diamètre 0,15 mm et de fils en polymère non résorbable en polypropylène de diamètre 0,1 mm.

Dans le cas de la présente invention, les dimensions de la plaque sont uniquement limitées par son utilisation chirurgicale, puisqu'elle doit pouvoir lorsqu'elle est roulée, passer à l'intérieur d'un trocart ayant en général 1 cm ou 1,2 cm de diamètre. En général des dimensions de plaque dans la gamme de 13 * 76 mm à 31 * 41 cm, avantageusement 15 * 15 cm, 15 * 13 cm ou 13 * 13 cm, en particulier 15 * 15 cm, avec une épaisseur de 0,3 à 0,8 mm, en particulier d'environ 0,45 mm, sont considérées comme désirables pour la facilité de manipulation chirurgicale. Par ailleurs, la plaque peut avoir toute géométrie nécessaire à son utilisation chirurgicale, qu'elle soit à contour rectangulaire, carré, triangulaire, circulaires ou ovale, la seule condition étant qu'elle puisse s'enrouler sur elle-même.

Avantageusement, la plaque textile selon la présente invention possède une résistance à la rupture supérieure à 16 N/cm pour les plaques destinées aux petites hernies inguinales, en particulier supérieure à 32 N/cm, pour les plaques destinée aux éventrations abdominales. Cette résistance est mesurée selon la norme l'ASTMD3787 suivant le test de Burst Strenght. Ce test consiste à utiliser une bille qui vient déformer la plaque textile tendu sur un anneau jusqu'à rupture. Le diamètre d'anneau est de 1 pouce (25,4 cm), le diamètre de la bille est de 0,38 inch (9,65 mm) pour une vitesse de test de 12 inchs/minutes (304,8 mm/minute). La résistance obtenue est divisée par le périmètre de l'anneau pour obtenir la résistance en N/cm. Dans un mode de réalisation particulièrement avantageux, la plaque textile selon la présente invention consiste en un tricot de type tulle de 28 g/m² réalisé avec un monofilament polypropylène de diamètre 0,1 mm.

Dans un autre mode de réalisation selon la présente invention, les moyens de déploiement sont disposés sur la face envers et/ou la face endroit de la plaque selon la présente invention. Avantageusement, dans la position au repos, la plaque selon la présente invention est enroulée sur elle-même de la face endroit vers la face envers selon la direction (P) et les moyens de déploiement sont disposés sur la face envers.

Avantageusement, les moyens de déploiement comprenant un polymère déshydraté apte à former un gel en milieu aqueux, le polymère n'est pas sous la forme d'un gel avant implantation. Il est donc déshydraté. Au sens de la présente invention, on entend par « polymère déshydraté » un polymère contenant moins de 1 % en poids d'eau par rapport au poids total du polymère. Après implantation, le milieu d'implantation étant un milieu aqueux, le polymère absorbe l'eau du milieu aqueux et se transforme en gel. Avantageusement, ce polymère ne forme pas de gel pour un taux d'humidité relative ambiante inférieure ou égale à 65 % d'humidité relative (HR). Avantageusement le polymère absorbe au moins 20 % en poids d'eau par rapport au poids total du polymère pour former un gel lors de son implantation.

Au sens de la présente invention, on entend par « gel » tout matériau constitué par un réseau polymérique retenant un liquide. Les gels possèdent une propriété commune avec les solides, celle de ne pas s'écouler sous son propre poids mais aussi une caractéristique des liquides, celle de se déformer sous l'effet d'une certaine contrainte. En quelque sorte, on peut assimiler "l'état gel" comme l'intermédiaire entre l'état liquide et l'état solide. Dans le cadre de la présente invention, le liquide retenu par le réseau polymère est une solution aqueuse, en particulier de l'eau.

En particulier, les moyens de déploiement du dispositif implantable selon la présente invention sont disposés sur la face envers et/ou la face endroit de la plaque et comprennent un polymère déshydraté apte à former un gel en milieu aqueux.

Avantageusement le polymère déshydraté est apte à former un gel en milieu aqueux à la température corporelle, c'est-à-dire d'environ 36 - 37°C. Ainsi, dans ce mode de réalisation particulier, le polymère ne forme pas de gel à une température inférieure à 35°C, avantageusement inférieure ou égale à 30°C et en particulier à une température proche de la température ambiante c'est-à-dire à 20 - 25°C.

De façon avantageuse, le gel formé par le polymère selon la présente invention peut être soluble ou insoluble en milieu aqueux, en particulier dans le milieu aqueux d'implantation. Ainsi, dans le cas où le gel est soluble en milieu aqueux, il peut se dissoudre en quelques heures, avantageusement en au moins 1 heure, une fois implanté.

De façon avantageuse, le polymère déshydraté apte à former un gel en milieu aqueux est tel que le gel obtenu à une teneur en eau d'au moins 20 % en poids lorsqu'il est totalement hydraté à 37°C, de manière avantageuse d'au moins 30% en poids, en particulier comprise entre 75 et 99 % en poids.

Dans une mode de réalisation particulier, le polymère déshydraté forme un gel au plus tard dans les 15 minutes de sa mise en contact avec un milieu aqueux, c'est-à-dire dans le cas de la présente invention au plus tard dans les 15 minutes de son implantation.

De façon avantageuse, le polymère déshydraté apte à former un gel en milieu aqueux peut être naturel ou synthétique. De façon encore plus avantageuse, ce polymère est bio résorbable, c'est-à-dire qu'il ne reste pas de façon permanente dans le corps humain. Avantageusement la résorption est relativement rapide, c'est-à-dire qu'elle a lieu dans un délai inférieur à trois mois, de façon à limiter les phénomènes inflammatoires.

Le polymère selon la présente invention doit être biocompatible puisqu'il est destiné à être implanté dans le corps humain.

Avantageusement, ledit polymère déshydraté apte à former un gel en milieu aqueux est choisi seul ou en combinaison parmi les polymères suivants : un dérivé de polysaccharide réticulé ou non, tels que le collagène, un dérivé d'acide hyaluronique ou la carboxyméthylcellulose ou un de ses dérivés, les composés vinyliques tels que la polyvinylpyrrolidone (PVP) ou la polyvinylpolypyrrolidone (crospovidone), les polyéthylène glycol, les copolymères d'acide lactique et de polyéthylèneglycol (PLLA/PEG), les copolymères d'acide lactique, d'acide glycolique et de polyéthylèneglycol (PLGA/PEG), les alcools polyvinyliques (PVA) et les polyacrylates tel que le polyhydroxyéthylméthacrylate (PHEM).

Au sens de la présente invention, on entend par dérivé de polysaccharide, aussi bien le polysaccharide considéré à l'état pur que ce dernier modifié chimiquement ou mélangé à d'autres produits ou adjuvant biocompatibles. En particulier le dérivé de polysaccharide est choisi dans le groupe constitué du collagène et de ses dérivés, des mucopolysaccharides, des polysaccharides polyanioniques, des glycosaminoglycanes, des celluloses modifiées et des mélanges de ceux-ci. Avantageusement le dérivé de polysaccharide est choisi dans le groupe constitué des dérivés de l'acide hyaluronique (HA) ou d'un de ses sels, des dérivés de la carboxyméthylcellulose (CMC) ou d'un de ses sels, des dérivés de la carboxyméthylamylose (CMA), des dérivés de l'hydroxypropylcellulose (HPC), des dérivés de l'hydroxypropylméthylcellulose (HPMC), des dérivés de la méthylcellulose (MC), des dérivés de l'hydroxyéthylcellulose (HEC), des dérivés de l'éthylcellulose (EC) ou un mélange de ceux-ci. Avantageusement il s'agit de la carboxyméthylcellulose (CMC).

Avantageusement, le dérivé de polysaccharide est un polymère ou un copolymère de cyclodextrine comprenant au moins un motif de base choisi dans le groupe constitué de l'alpha-cyclodextrine, de la beta-cyclodextrine, de la gamma-cyclodextrine et leurs mélanges ou les dérivés aminés, méthylés, hydroxypropylés, sulfobutylés, carboxyméthylés ou carboxyliques de ces cyclodextrines et leurs mélanges.

L'acide hyaluronique est un mucopolysaccharide naturel présent entre autres dans le fluide synovial et dans les parois des vaisseaux sanguins. Ce polysaccharide consiste en des résidus de N-acétyle-D-glucosamine et d'acide D-glucuronique relié par des liaisons β 1-3-glucuronidiques et β 1-4-glucosaminidique respectivement, de sorte que l'unité de construction est désignée -(1>4)-β-D-GlcA-(1>3)-β-D-GlcNac. Les sels de l'acide hyaluronique sont en particulier choisis parmi l'hyaluronate de sodium, l'hyaluronate de potassium, l'hyaluronate de magnésium et l'hyaluronate de calcium. Le dérivé d'acide hyaluronique le plus avantageux est insoluble dans l'eau et biocompatible. Il peut être obtenu par exemple en faisant réagir l'acide hyaluronique avec un agent réticulant polyfonctionnel tel que par exemple un époxyde polyfonctionnel. Avantageusement les procédés pour obtenir de tels produits sont décrits dans les demandes de brevet WO 89/02445, WO 92/00105 et WO 92/20349.

En particulier les alcools polyvinyliques utilisables sont ceux décrits dans le brevet US 5.192.326.

Les polyacrylates peuvent en particulier être obtenus par polymérisation de l'acide acrylique, de l'acide méthacrylique, du 2-hydroxyléthylméthacrylate, de l'octyle acrylate, du 2-éthylhexyle acrylate, de l'isooctyle acrylate, de l'isononyle acrylate, de l'hexyle acrylate, du butyle acrylate et copolymérisation de ces monomères entre eux ou avec la N-vinyle pyrrolidone, les N-vinyle lactames, l'acrylamide, les polyuréthanes, le polyacrylonitrile, l'acide P-acryloyloxypropionique, l'acide vinylphosphonique. En particulier il s'agit du polyhydroxyéthylméthacrylate (PHEM).

Les composés vinyliques peuvent en particulier être choisis parmi les polymères obtenus par polymérisation de N-vinylpyrrolidone, N-vinyle lactame, hydroxyéthylcellulose vinyle, vinyle carbonate, vinyle trifluoacétate et des monomères vinyliques. Avantageusement il s'agit de la polyvinylpyrrolidone.

Dans un mode de réalisation particulièrement avantageux de la présente invention, les moyens de déploiement consiste en de la polyvinylpyrrolidone, qui est présente sous forme déshydratée avant implantation, et qui sur le site de l'implantation s'hydrate pour former un gel.

Le polymère déshydraté selon la présente invention peut être utilisé seul ou en mélange avec un plastifiant ce qui permet de faciliter la pose du polymère sur la plaque selon la présente invention. On entend par « plastifiant » au sens de la présente invention, toute substance, autres que les molécules d'eau, apte notamment à diminuer la température de transition vitreuse du polymère selon la présente invention. Avantageusement le plastifiant est choisi parmi les polyalcools tels que le polyéthylène glycol (PEG), en particulier dans le cas où le polymère déshydraté consiste en du PVP ou de la CMC. De façon avantageuse la teneur en plastifiant du polymère déshydraté selon la présente invention est inférieure à 4 % poids par rapport au poids total du polymère déshydraté, en particulier inférieur à 2 %, de préférence d'au moins 0,5 %, en particulier d'environ 1 %. Avantageusement, le plastifiant a une masse moléculaire moyenne en poids Mw comprise entre 100 g /mol et 700 g/mol.

Dans un autre mode de réalisation de la présente invention, le polymère déshydraté apte à former un gel en milieu aqueux est un polymère bio-adhésif dont la fonction adhésive est activable en milieu aqueux. En particulier ce polymère bioadhésif est celui décrit dans les demandes de brevet FR 2.920.671 et WO 2005/058383. De façon avantageuse il s'agit du PVP, de la CMC ou de polyacrylates, en particulier du PVP ou de la CMC, de façon encore plus avantageuse du PVP. De préférence, le polymère bioadhésif à une masse moléculaire moyenne en poids Mw compris entre 44 000g /mol et 2.10⁶ g/mol. Avantageusement ce polymère bioadhésif est plastifié à l'aide d'un plastifiant comme indiqué ci-dessus, en particulier à l'aide de polyéthylène glycol. En particulier il s'agit de PVP (tel que le Kollidon K90 de la société BASF) plastifié par 1 % de PEG (tel que le Lutrol E400 de la société BASF). Ainsi dans le cadre de ce mode de réalisation particulier de la présente invention, le polymère déshydraté joue à la fois le rôle de moyens de déploiement et d'adhésif chirurgical dans le milieu d'implantation, grâce à l'activation de ces deux fonctions à l'aide du milieu aqueux d'implantation. Dans le cadre de ce mode de réalisation, le dispositif peut comporter un film de protection disposée sur la ou les faces comportant le polymère bioadhésif afin de protéger ce polymère et, dans le cas où seule une face contient ce polymère, de permettre au chirurgien de savoir laquelle. Ce film de protection peut en particulier être un tissu tissé, non-tissé ou tricoté. Ce film de protection sera retiré par le chirurgien avant implantation du dispositif selon la présente invention.

Dans encore un autre mode de réalisation de la présente invention, le moyen de déploiement est disposé sur une seule face de la plaque et forme un film continu non poreux de polymère déshydraté recouvrant de façon uniforme toute la surface de cette face. Ainsi, dans ce cas le moyen de déploiement peut également jouer le rôle de moyens permettant d'éviter les adhérences post chirurgicales après implantation du dispositif dans le corps humain. En effet, l'absence de porosité empêche la colonisation cellulaire immédiate post chirurgicales du côté du dispositif comportant ce film. De façon avantageuse ce film est lisse. De façon encore plus avantageuse, il déborde de la surface de la plaque selon la présente invention de façon à protéger le dispositif de contacts viscéraux, les bords pouvant être par exemple de 5 à 10 mm. En particulier ce type de films est décrit dans les demandes de brevet WO 99/06079 et WO 96/08277. De façon avantageuse le polymère déshydraté utilisé est du collagène ou un de ses dérivés ou un dérivé de l'acide hyaluronique. Dans le cadre de ce mode de réalisation, si l'on souhaite que le moyen de déploiement ait également une fonction anti-adhérence post chirurgicale, le polymère déshydraté utilisé ne doit pas être hydrosoluble et ne doit pas se résorber pendant au moins 28 jours.

Dans un autre mode de réalisation de présente invention, les moyens de déploiement comprennent un revêtement à base dudit polymère recouvrant tout ou partie de la face envers et/ou la face endroit et dont la masse surfacique (g/m²) avant implantation, c'est-à-dire avant la formation du gel, représente au moins trois fois la masse surfacique (g/m²) de la plaque textile, avantageusement au moins quatre fois. En particulier l'épaisseur de ce revêtement est comprise entre 0,1 et 0,3 mm. De façon encore plus avantageuse, le dispositif (plaque + polymère déshydraté) présente une masse surfacique comprise entre 160 et 240 g/ m².

Dans un mode de réalisation particulièrement avantageux, la plaque textile selon la présente invention est enduite de 130 g/m² de polyvinylpyrrolidone déshydratée.

Le dispositif selon la présente invention peut être préparé par un procédé bien connu de l'homme du métier. En particulier lorsque les moyens de déploiement comprennent un polymère apte à former un gel en milieu aqueux, le dispositif est obtenu par enduction ou imprégnation de la plaque textile selon la présente invention par ledit polymère, par des moyens bien connus de l'homme du métier, ou encore par collage par laminage, par fusion ou par une colle biocompatible d'un film de polymère selon la présente invention sur la plaque textile selon la présente invention, par des moyens bien connus de l'homme du métier. Enfin ledit polymère peut également être pulvérisé sur la surface de la plaque textile selon la présente invention. L'enduction, la pulvérisation, le collage ou l'imprégnation peuvent être réalisés sur la face envers et/ou endroit de la plaque textile selon la présente invention, avantageusement sur la face envers. L'imprégnation peut être réalisée en plongeant la plaque textile dans un bain contenant le polymère puis déshydratation du produit obtenu. L'enduction peut être réalisée de la façon suivante : on dépose un bandeau à partir d'une solution aqueuse contenant 2260 grammes de Kollidon 90 de la société BASF (polyvinylpyrrolidone), 25 grammes de Lutrol E400 de la société BASF (polyéthylèneglycol), et 5118 gramme d'eau distillée sur une face de la plaque textile. On utilise ensuite une racle afin d'enduire toute la face de la plaque textile. Grâce à cette racle, le polymère va pénétrer totalement à l'intérieur de la plaque textile de façon à ressortir de l'autre côté de la plaque sur la deuxième face. Ainsi le polymère sera présent sur la deuxième face et sera absent de la première face sur laquelle la racle est passée. Le séchage dans le cas de l'enduction, de la pulvérisation ou de l'imprégnation est avantageusement réalisé à plat, en particulier à température ambiante avantageusement pendant quelques heures, avantageusement au moins 4 heure dans le cas d'une enduction comme indiquée ci-dessus, à une hygrométrie d'environ 65 % d'humidité relative pour éviter le gondolement de la plaque. Le séchage et donc lent. Le dispositif est ensuite découpé selon les dimensions carrés (15 cm x 15 cm ou 30 cm x 30 cm), rectangulaire (15 cm x 20 cm ou 12 cm x 15 cm), ou elliptique (de demi grand-axe 7,5 cm et de demi petit-axe 5,5 cm) puis enroulé sur lui-même dans une configuration de tube, avantageusement de façon à ce que les moyens de déploiement se trouvent situés vers l'intérieur du dispositif après enroulement. Le dispositif est maintenu enroulé pendant au moins une semaine afin de conserver une position au repos enroulé sur lui-même dans une configuration de tube. Il peut ensuite être conditionné puis stérilisé, avant utilisation dans le cadre d'une chirurgie par coelioscopie. Avantageusement la stérilisation a lieu avec de l'oxyde d'éthylène. Ces techniques de stérilisation connue élèvent la température à environ 60°C et tuent les germes. La stérilisation peut également être effectuée à l'aide de rayons gamma mais à condition que la plaque textile ne soit pas en polypropylène. En effet le polypropylène est dégradé si une telle stérilisation et utilisée.

Le dispositif selon la présente invention peut donc être utilisé en chirurgie par voie coelioscopie, en particulier en chirurgie pariétale. En particulier le dispositif selon la présente invention peut servir de prothèse, par exemple pour remplacer le péritoine, pour la réfection de la paroi abdominale, destinée au traitement de hernie ou d'éventration, ou pour tout autre chirurgie nécessitant la pose d'une prothèse à base d'une plaque de textile souple selon la présente invention de conformation sensiblement plane. Le dispositif selon la présente invention peut également être utilisé en urologie, en particulier pour le traitement du prolapsus par voie haute par coelioscopie.

En particulier, le procédé chirurgical consiste en l'introduction dans la cavité abdominale par le chirurgien de la plaque pré-enroulée (dispositif selon la présente invention) à travers un des trocarts qui traverse la paroi abdominale. Sur le site d'implantation, à la sortie du trocart, la plaque se déploie d'elle-même à l'aide des moyens de déploiement et en particulier grâce à la formation du gel, qui en raison de son poids va donner à la plaque sa forme sensiblement plane. Le chirurgien peut ensuite la fixer facilement à la paroi dans cette conformation déployée. Il n'a donc pas besoin d'effectuer de manipulation supplémentaire pour dérouler la plaque à la sortie du trocart.

La présente invention concerne en outre un kit tel que décrit dans les revendications comprenant un tube principal ayant un volume intérieur déterminé, un dispositif implantable selon la présente invention, et une pince de préhension ayant deux tiges, ladite plaque textile selon la présente invention étant dans sa position au repos enroulée sur elle-même disposée autour d'au moins une tige et logée dans le volume intérieur du tube principal.

Dans un mode de réalisation particulier de la présente invention, le kit comprend un moyen d'assemblage amovible des extrémités distales desdites deux tiges, ces extrémités étant donc indépendantes l'une de l'autre. Avantageusement le moyen d'assemblage des extrémités distales consiste en un capuchon en silicone. De façon avantageuse les deux tiges ne sont liées que par les extrémités proximales. Avantageusement le moyen d'assemblage des extrémités proximales consiste en un point de soudure, une vis, de la colle ou une gaine thermo-rétractable.

Dans un autre mode de réalisation de la présente invention, la pince de préhension comporte une partie courbée pour faciliter la préhension. Avantageusement, cette partie courbée se trouve à l'extrémité proximale de l'une des deux tiges.

Dans encore un autre mode de réalisation de la présente invention, le dispositif implantable selon l'invention a une extrémité insérée entre les deux tiges, le dispositif étant enroulée sur lui-même autour des deux tiges de façon à ce que la deuxième extrémité se trouve à l'extérieur des deux tiges.

Avantageusement, le tube du kit selon la présente invention permet le maintien de la forme enroulée et donc de calibrer le diamètre externe du dispositif selon la présente invention.

Le kit selon la présente invention peut être fabriqué par des méthodes bien connues de l'homme du métier. En particulier le dispositif selon la présente invention est inséré dans le kit avant sa stérilisation.

Le kit selon la présente invention peut être utilisé par le chirurgien de la façon suivante en cas de réparation pariétale par voie coelioscopie : le chirurgien retire le tube. Il garde la pince de préhension sur laquelle est enroulé le dispositif implantable selon l'invention et se débarrasse du tube. Il retire ensuite le moyen d'assemblage amovible des extrémités distales des tiges. Le dispositif implantable selon la présente invention qui est toujours enroulé sur les tiges de la pince de préhension, est alors introduit dans l'un des trocarts. Dès que le dispositif est dans la cavité abdominale, le chirurgien retire la pince de préhension pour laisser tomber le dispositif sur les viscères. Il introduit ensuite ces deux pinces dans les trocarts pour positionner le dispositif sur la partie de la paroi présentant le défaut de paroi abdominale. Le dispositif se déploie alors spontanément pour retrouver une forme sensiblement plane. Il peut alors fixer facilement le dispositif.

L'invention sera mieux comprise à la lecture de la description et des dessins qui suivent :
- la figure 1 montre une perspective en vue éclatée d'un mode de réalisation du kit selon la présente invention ;
- la figure 2 illustre le dispositif implantable selon l'invention avant implantation ;
- la figure 3 illustre un exemple de kit selon la présente invention ;
- la figure 4 est une représentation schématique d'une coupe transversale d'un premier exemple de dispositif selon la présente invention schématisé dans l'organisme après déploiement pour la réfection de hernie en intra-abdominale extra péritonéal ;
- la figure 5 est une représentation schématique d'une coupe transversale d'un deuxième exemple de dispositif selon la présente invention schématisé dans l'organisme après déploiement pour la réfection de hernie en intra-abdominale intra péritonéal.

Ainsi, selon un exemple de réalisation de la présente invention, il est fourni un dispositif implantable 1 comprenant une plaque textile 2 ayant des faces envers 3 et endroit 4 opposées. Cette plaque comprend des orifices de forme géométrique déterminée 6. Elle est pourvue de moyens de déploiement activable par milieu aqueux 5 sur sa face envers 3 qui consiste en une couche de polymère apte à former un gel en milieu aqueux. Avantageusement il s'agit d'un tricot de type tulle de 28 g/m² réalisé avec un monofilament polypropylène de diamètre 0,1 mm enduit de 130 g/m² de polyvinylpyrrolidone déshydratée plastifié avec 1 % de polyéthylèneglycol.

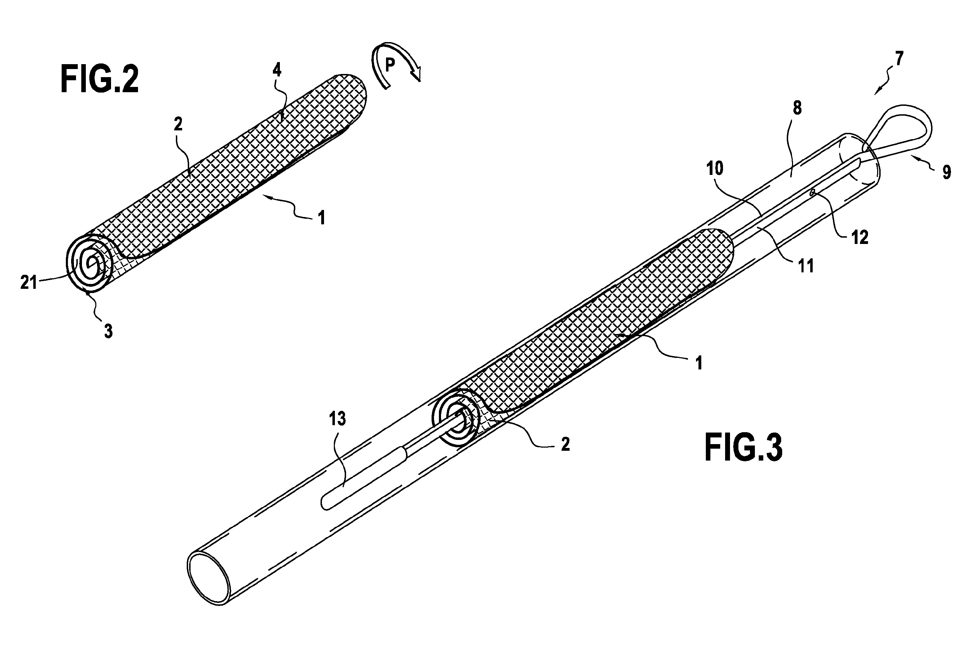

Comme illustré à la figure 2, ce dispositif est fourni au chirurgien sous la forme pré-enroulée dans la direction P avant son implantation.

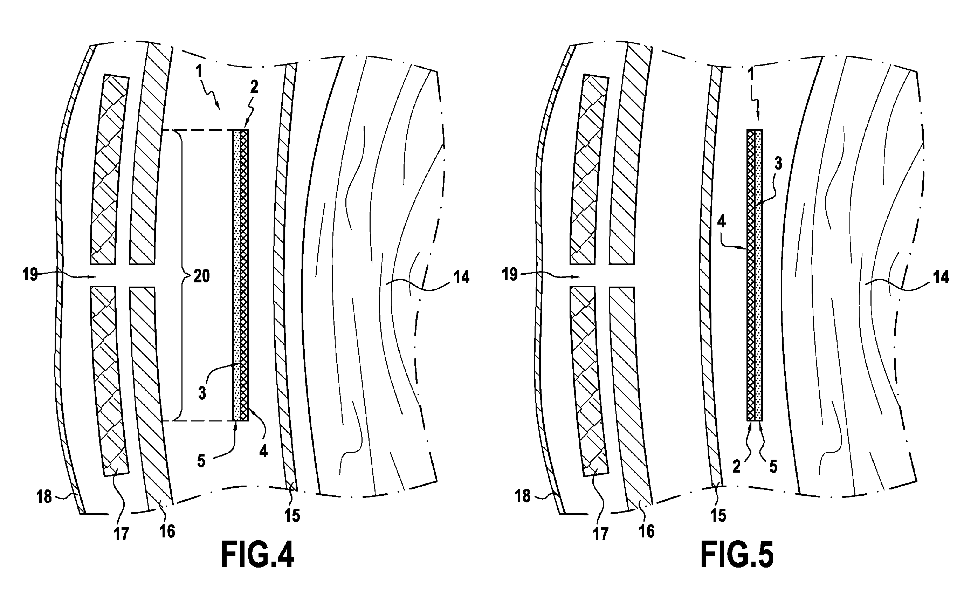

Après implantation, le dispositif retrouvera une forme sensiblement plane comme illustré aux figures 4 et 5.

Selon un autre exemple de réalisation de la présente invention illustré aux figures 1 et 3, il est fourni un kit 7 comprenant un tube principal 8 de volume intérieur déterminé, le dispositif selon l'invention 1 par exemple tel que décrit ci-dessus, étant enroulé autour de deux tiges 10 et 11 d'une pince de préhension 9. Une des deux tiges 10 est courbée dans sa partie proximale pour une préhension plus aisée. Les deux tiges 10 et 11 sont fixées l'une à l'autre à l'extrémité proximale à l'aide d'un moyen de fixation 12 et à l'extrémité distale à l'aide d'un moyen de fixation amovible 13, par exemple un capuchon en silicone 13.

Le dispositif implantable 1 selon la présente invention peut être implanté en intraabdominale extra péritonéal comme schématisé à la figure 4. Il est donc disposé entre les viscères 14 et le péritoine 15 d'un côté et la paroi abdominale 16, une couche de tissu adipeux 17 et la peau 18 de l'autre côté. Dans l'exemple représenté sur la figure 4, le moyen de déploiement activable par milieu aqueux 5 consiste en un polymère déshydraté qui a également la fonction d'être bioadhésif et qui se trouve disposé sur une seule face 3 de la plaque textile 2. Cette face 3 est disposée au regard de l'orifice herniaire ou de l'éventration 19, obturant la couche de tissu adipeux 17 et la paroi abdominale 16, après que le sac herniaire ait été disséqué et repoussé (non représenté). Après implantation, une fois que la fonction de déploiement et la fonction adhésive sont activées, le dispositif 1 adhère immédiatement à la zone 20. Le pouvoir adhésif et la quantité de polymère bioadhésif sont suffisants pour que le dispositif selon la présente invention ne s'écroule pas sur lui-même et que toute la surface de la face 3 reste en contact avec la zone 20 à renforcer suffisamment longtemps pour que la fibrose et le développement des tissus conjonctifs aient lieu et fixent définitivement à la plaque textile 2. Au bout de cette période d'environ 28 jours, le polymère bioadhésif 5 a été totalement absorbé et éliminé naturellement par l'organisme de sorte que seule la plaque textile 2 ayant une masse surfacique faible subsiste et assure le rôle de renfort mécanique de la zone 20. Dans cet exemple, avant implantation, le dispositif selon la présente invention sera muni d'une feuille de protection 21 comme illustré à la figure 1 qui permet de protéger les moyens de déploiement 5, en particulier lorsqu'ils ont également la fonction de moyen d'adhésion, et qui servent au chirurgien à reconnaître facilement sur quelle face 3 de la plaque textile 2 se trouvent ces moyens.

Le dispositif implantable 1 selon la présente invention peut également être implanté en intraabdominale intra péritonéal comme schématisé à la figure 5. Il est donc disposé entre d'un côté les viscères 14 et de l'autre côté le péritoine 15, la paroi abdominale 16, une couche de tissu adipeux 17 et la peau 18, au regard de l'éventration ou de l'orifice herniaire 19 une fois que le sac herniaire a été repoussé (non représenté). Dans l'exemple représenté sur la figure 5, le moyen de déploiement activable par milieu aqueux 5 consiste en un film polymère qui a également la fonction d'éviter les adhérences post chirurgicales et qui se trouve disposé sur une seule face 3 de la plaque textile 2. Cette face 3 est donc disposée au regard des viscères 14 afin d'éviter l'adhérence des viscères sur la plaque textile 2. En effet, les viscères 14 ne peuvent s'accrocher à la plaque textile 2 notamment par la fibrose. En général, ce film va se résorber mais pendant une durée compatible avec la reconstitution tissulaire, par exemple celle du péritoine. Sa résorption assure la protection contre les phénomènes d'adhésion initiaux, c'est-à-dire dans la première semaine postopératoire, ou autrement dit, pendant le laps de temps nécessaire à l'intégration tissulaire de la face opposée 4 de la plaque textile 2.

Ainsi grâce au dispositif selon la présente invention, le chirurgien n'est plus obligé d'enrouler le dispositif sur le site opératoire. En outre, il peut utiliser une plaque légère (inférieure à 50 g/m²) et la plaque ne possède pas suffisamment de mémoire de forme pour se dérouler difficilement. Au contraire le dispositif selon la présente invention se déroulera spontanément après implantation par chirurgie coelioscopie par exemple dans les conditions de température et d'humidité intraabdominale (37°C et 100 % d'humidité relative) pour reprendre alors une forme sensiblement plane. Avantageusement, il est en revanche impossible de le dérouler sans exercer une contrainte relativement importante dans les conditions de taux d'humidité relative ambiante inférieure de 65 %, et en particulier à une température ambiante inférieure à 30°C.

## Revendications

1. Dispositif implantable (1), notamment par coelioscopie, comprenant une plaque textile (2) ayant des faces envers (3) et endroit (4) opposées, une masse surfacique inférieure ou égale à 50 g/m² et comprenant des monofilaments, ladite plaque se présentant dans une position au repos enroulée sur elle-même dans une configuration de tube, comprenant des moyens de déploiement (5) activables par milieu aqueux aptes à faire passer ladite plaque de ladite position au repos à une position d'implantation dans laquelle ladite plaque a une configuration sensiblement plane, lesdits moyens de déploiement (5) étant disposés sur la face envers (3) et/ou la face endroit (4) de la plaque (2) et comprenant un polymère déshydraté apte à former un gel en milieu aqueux.

2. Dispositif implantable selon la revendication 1, **caractérisé en ce que** dans la position au repos, la plaque (2) est enroulée sur elle-même de la face endroit (4) vers la face envers (3) selon la direction (P) et **en ce que** les moyens de déploiement (5) sont disposés sur la face envers (3).

3. Dispositif selon l'une ou l'autre des revendications 1 à 2, **caractérisé en ce que** la plaque (2) comprend des orifices (6) ayant une forme géométrique déterminée, notamment une forme de losange, rectangle, carré, hexagonale, ou octogonale, délimitée par des segments constitués de fils tricotés.

4. Dispositif selon la revendication 3, **caractérisé en ce que** les droites qui prolongent lesdits segments sont sécantes à la direction d'enroulement (P) de ladite plaque.

5. Dispositif selon l'une quelconque des revendications 1 à 4, **caractérisé en ce que** ledit polymère déshydraté apte à former un gel en milieu aqueux est choisi seul ou en combinaison parmi les polymères suivants : un dérivé de polysaccharide réticulé ou non tels que le collagène, un dérivé d'acide hyaluronique ou la carboxyméthylcellulose ou un de ses dérivés, les composés vinyliques tels que la polyvinylpyrrolidone (PVP) ou la polyvinylpolypyrrolidone (crospovidone), les polyéthylène glycol, les copolymères d'acide lactique, et de polyéthylèneglycol (PLLA/PEG), les copolymères d'acide lactique d'acide glycolique et de polyéthylèneglycol (PLGA/PEG), les alcools polyvinylique (PVA) et les polyacrylates tel que le polyhydroxyéthylméthacrylate (PHEM).

6. Dispositif selon la revendication 5, **caractérisé en ce que** ledit polymère déshydraté est la polyvinylpyrrolidone (PVP).

7. Dispositif selon l'une quelconque des revendications 1 à 6, **caractérisé en ce que** ledit polymère déshydraté est un polymère bio-adhésif dont la fonction adhésive est activable en milieu aqueux.

8. Dispositif selon l'une quelconque des revendications 1 à 7, **caractérisé en ce que** le moyen de déploiement est disposé sur une seule face (3) de la plaque (2) et forme un film continu non poreux de polymère déshydraté recouvrant de façon uniforme toute la surface de cette face (3).

9. Dispositif selon l'une quelconque des revendications 1 à 8, **caractérisé en ce que** les moyens de déploiement (5) comprennent un revêtement à base dudit polymère recouvrant tout ou partie la face envers (3) et/ou la face endroit (4) dont la masse surfacique (g/m²) représente au moins trois fois la masse surfacique (g/m²) de la plaque textile (2).

10. Dispositif selon l'une quelconque des revendications 1 à 9, **caractérisé en ce que** la plaque textile (2) est tricotée avec des monofilaments, de préférence ayant un diamètre inférieur ou égale à 0,2 mm.

11. Kit (7) comprenant un tube principal (8) ayant un volume intérieur déterminé, un dispositif (1) selon l'une quelconque des revendications 1 à 10, et une pince de préhension (9) ayant deux tiges (10, 11), ladite plaque (2) étant dans sa position au repos enroulée sur elle-même disposée autour d'au moins une tige (10,11) et logée dans le volume intérieure du tube principal (8).

12. Kit selon la revendication 11, **caractérisé en ce qu'**il comprend un moyen d'assemblage amovible (13) des extrémités distales desdites deux tiges (10,11).

## Patentansprüche

1. Implantierbare Vorrichtung (1), insbesondere mittels Zölioskopie, umfassend eine Textilscheibe (2) mit gegenüberliegenden linken (3) und rechten (4) Seiten, einer flächenbezogenen Masse von weniger als oder gleich 50 g/m² und Monofilamenten, wobei die Scheibe in einer Ruhestellung auf sich selbst aufgerollt in einer Röhrenausführung vorliegt, mit durch wässriges Medium aktivierbaren Entfaltungsmitteln (5), die geeignet sind, die Scheibe von der Ruhestellung in eine Implantationsstellung zu überführen, in der die Scheibe eine im Wesentlichen ebene Ausführung aufweist, wobei die Entfaltungsmittel (5) auf der linken Seite (3) und/oder der rechten Seite (4) der Scheibe (2) angeordnet sind und ein Trockenpolymer umfassen, das geeignet ist, in wässrigem Medium ein Gel zu bilden.

2. Implantierbare Vorrichtung nach Anspruch 1, **dadurch gekennzeichnet, dass** die Scheibe (2) in der Ruhestellung von der rechten Seite (4) zur linken Seite (3) in der Richtung (P) auf sich selbst aufgerollt ist und dass die Entfaltungsmittel (5) auf der linken Seite (3) angeordnet sind.

3. Vorrichtung nach dem einen oder dem anderen der Ansprüche 1 bis 2, **dadurch gekennzeichnet, dass** die Scheibe (2) Öffnungen (6) mit einer bestimmten geometrischen Form, insbesondere einer Rauten-, Rechteck-, Quadrat-, Sechseck- oder Achteckform, welche durch von gestrickten Fäden gebildete Segmente begrenzt ist, umfasst.

4. Vorrichtung nach Anspruch 3, **dadurch gekennzeichnet, dass** die Geraden, welche die Segmente fortsetzen, die Aufrollrichtung (P) der Scheibe schneiden.

5. Vorrichtung nach einem der Ansprüche 1 bis 4, **dadurch gekennzeichnet, dass** das Trockenpolymer, welches geeignet ist, in wässrigem Medium ein Gel zu bilden, allein oder in Kombination aus den folgenden Polymeren ausgewählt ist: einem vernetzten oder nicht vernetzten Polysaccharid-Derivat, wie Kollagen, einem Hyaluronsäure-Derivat oder Carboxymethylcellulose oder einem seiner Derivate, den Vinylverbindungen, wie Polyvinylpyrrolidon (PVP) oder Polyvinylpolypyrrolidon (Crospovidon), Polyethylenglykol, den Copolymeren von Milchsäure und von Polyethylenglycol (PLLA/PEG), den Copolymeren von Milchsäure-Glykolsäure und von Polyethylenglycol (PLGA/PEG), den Polyvinylalkoholen (PVA) und den Polyacrylaten, wie Polyhydroxyethylmethacrylat (PHEM).

6. Vorrichtung nach Anspruch 5, **dadurch gekennzeichnet, dass** das Trockenpolymer Polyvinylpyrrolidon (PVP) ist.

7. Vorrichtung nach einem der Ansprüche 1 bis 6, **dadurch gekennzeichnet, dass** das Trockenpolymer ein bioadhäsives Polymer ist, dessen Haftfunktion in wässrigem Medium aktivierbar ist.

8. Vorrichtung nach einem der Ansprüche 1 bis 7, **dadurch gekennzeichnet, dass** das Entfaltungsmittel auf einer einzigen Seite (3) der Scheibe (2) angeordnet ist und einen durchgehenden, nicht porösen Film aus Trockenpolymer bildet, der die gesamte Oberfläche dieser Seite (3) gleichmäßig bedeckt.

9. Vorrichtung nach einem der Ansprüche 1 bis 8, **dadurch gekennzeichnet, dass** die Entfaltungsmittel (5) eine Beschichtung auf der Basis des Polymers umfassen, welche die gesamte linke Seite (3) und/oder rechte Seite (4) oder einen Teil dieser bedeckt, deren flächenbezogene Masse (g/m²) wenigstens das Dreifache der flächenbezogenen Masse (g/m²) der Textilscheibe (2) ausmacht.

10. Vorrichtung nach einem der Ansprüche 1 bis 9, **dadurch gekennzeichnet, dass** die Textilscheibe (2) mit Monofilamenten, vorzugsweise mit einem Durchmesser von unter oder gleich 0,2 mm, gestrickt ist.

11. Kit (7), umfassend eine Hauptröhre (8) mit einem bestimmten Innenvolumen, eine Vorrichtung (1) nach einem der Ansprüche 1 bis 10 und eine Greifzange (9) mit zwei Stangen (10, 11), wobei die Scheibe (2) in ihrer auf sich selbst aufgerollten Ruhestellung um wenigstens eine Stange (10, 11) herum angeordnet und in dem Innenvolumen der Hauptröhre (8) untergebracht ist.

12. Kit nach Anspruch 11, **dadurch gekennzeichnet, dass** er ein Mittel zum lösbaren Verbinden (13) der distalen Enden der beiden Stangen (10, 11) umfasst.

## Claims

1. An implantable device (1), particularly using laparoscopy, comprising a textile plate (2) having opposite reverse (3) and front (4) faces, a weight per unit surface area less than or equal to 50 g/m² and comprising monofilaments, said plate being in one position at rest coiled over itself in a tube configuration, comprising deployment means (5) which can be activated by aqueous medium, capable of causing said plate to change from said rest position into an implantation position wherein said plate has a substantially planar configuration, said deployment means (5) being positioned on the reverse face (3) and/or the front face (4) of the plate (2) and comprising a dehydrated polymer capable of forming a gel in an aqueous medium.

2. The implantable device according to claim 1, **characterized in that** in the rest position, the plate (2) is coiled over itself from the front face (4) toward the reverse face (3) in the direction (P) and **in that** the deployment means (5) are positioned on the reverse face (3).

3. The device according to one or the other of claims 1 to 2, **characterized in that** the plate (2) comprises openings (6) having a predetermined geometric shape, particularly a lozenge, rectangle square, hexagonal or octagonal shape, delimited by segments consisting of knitted yarns.

4. The device according to claim 3, **characterized in that** the straight lines which extend said segments are secant to the coiling direction (P) of said plate.

5. The device according to any one of claims 1 to 4, **characterized in that** said dehydrated polymer capable of forming a gel in an aqueous medium is selected alone or in combination among the following polymers: a polysaccharide derivative, cross-linked or not such as collagen, a hyaluronic acid derivative or carboxymethylcellulose or one of its derivatives, vinyl compounds such as polvinylpyrrolidone (PVP) or polyvinylpolypyrrolidone (crospovidone), polyethylene glycols, Poly(1-lactic acid) and polyethylene glycol (PLLA/PEG), the copolymers of poly(lactic-co-glycolic) acid and polyethylene glycol (PLGA/PEG), polyvinyl alcohols (PVA) and polyacrylates such as polyhydroxyethyl methacrylate (PHEM).

6. The device according to claim 5, **characterized in that** said dehydrated polymer is polyvinylpyrrolidone (PVP).

7. The device according to any one of claims 1 to 6, **characterized in that** said dehydrated polymer is a bioadhesive polymer the adhesive function whereof can be activated in an aqueous medium.

8. The device according to any one of claims 1 to 7, **characterized in that** the deployment means is positioned on a single face (3) of the plate (2) and forms a continuous nonporous film of dehydrated polymer covering uniformly all the surface of this face (3).

9. The device according to any one of claims 1 to 8, **characterized in that** the deployment means (5) comprise a covering based on said polymer covering all or part of the reverse face (3) and/or the front face (4) of which the mass per unit surface area (g/m2) represents at least three times the mass per unit surface area (g/m2) of the textile plate (2).

10. The device according to any one of claims 1 to 9, **characterized in that** the textile plate (2) is knitted from monofilaments, preferably having a diameter less than or equal to 0.2 mm.

11. A kit (7) comprising a main tube (8) having a predetermined inner volume, a device (1) according to any one of claims 1 to 10, and a gripper (9) having two shanks (10, 11), said plate (2) being, in its rest position, coiled over itself, positioned around at least one shank (10, 11) and housed in the inner volume of the main tube (8).

12. The kit according to claim 11, **characterized in that** it comprises a removable assembly means (13) for the distal ends of said two shanks (10, 11).
